# EUROPÄISCHE PATENTSCHRIFT

(11) **EP 1 255 497 B1**
(45) Veröffentlichungstag und Bekanntmachung des Hinweises auf die Patenterteilung: **23.06.2004**
(21) Anmeldenummer: 00969472.0
(22) Anmeldetag: 10.10.2000
(51) Int. Cl.: A61B 17/72

(54) **VERRIEGELUNGSNAGEL UND ZIELGERÄT**
LOCKING NAIL AND TARGET APPLIANCE
CLOU D'IMMOBILISATION ET APPAREIL DE VISEE

(30) Priorität: 19.02.2000 DE 20003053 U
(43) Veröffentlichungstag der Anmeldung: 13.11.2002
(73) Patentinhaber: Stryker Trauma GmbH, 24232 Schönkirchen (DE)
(72) Erfinder: ANASTOPOULOS, George, 16341 Athens (GR); ROBIONECK, Paul, Bernd, 24211 Preetz (DE)
(74) Vertreter: Graalfs, Edo, Dipl.-Ing.
(86) Internationale Anmeldenummer: PCT/EP2000/009935
(87) Internationale Veröffentlichungsnummer: WO 2001/060272

(56) Entgegenhaltungen:
- EP-A- 0 192 840
- EP-A- 0 355 411
- EP-A- 0 715 832
- DE-C- 19 703 987
- DE-U- 29 806 564
- US-A- 5 658 287

## Beschreibung

Die Erfindung bezieht sich auf ein Implantationssystem mit einem Verriegelungsnagel und einem Zielgerät nach Anspruch 1.

Verriegelungsnägel zur Versorgung von Frakturen von Röhrenknochen sind weit verbreitet. Ihre Anwendung ist etwa in "The Journal of Trauma" von 1993 Vol. 35 Nr. 5, Seiten 772 bis 775 beschrieben. Typisch für derartige Verriegelungsnägel ist die Anordnung von zwei Querbohrungen am distalen Ende und mindestens einer Querbohrung am proximalen Ende. Durch die Querbohrungen werden Knochenschrauben hindurchgeführt. Sie sind an gegenüberliegenden Seiten in die Kortikalis eingeschraubt. Dadurch ist der Verriegelungsnagel axial und gegen Drehung gesichert.

Ein Problem bei dem Einsatz derartiger Verriegelungsnägel besteht darin. die Lage der Querbohrungen zu identifizieren, um die Kortikalis von außen an der richtigen Stelle anzubohren. Es ist eine Reihe von Zielgeräten bekannt geworden. die mit Röntgenstrahlen arbeiten. um die Lage der Querbohrungen relativ zu einem Zielgerät zu identifizieren. Es ist daher möglich. mit Hilfe des Zielgerätes und einer sogenannten Bohr- oder Zielhülse den Knochen an der richtigen Stelle anzubohren. Die bekannten Zielgeräte werden zumeist fest mit dem proximalen Ende des Nagels verbunden. Auf diese Weise läßt sich bereits annähernd genau die Lage der Querbohrungen vorgeben. Allerdings ist zu berücksichtigen, daß durch die Krümmung des Knochens und durch die mögliche Torsion des Nagels beim Eintreiben die vermutete Lage der Querbohrungen nicht mit der tatsächlichen übereinstimmt.

Obwohl mit Hilfe der Röntgenstrahlen relativ genau die Lage der Querbohrungen ermittelt werden kann. ist der Einsatz von Röntgenstrahlen wegen der Beeinträchtigung von Patient und Chirurg nicht immer das Mittel der Wahl. Es ist daher auch bekannt geworden, Zielgeräte ohne Durchleuchtungsmittel anzuwenden. Wie schon erwähnt, ergibt sich die ungefähre axiale Lage der Querbohrungen bereits aus dem Abstand, den die Bohrungen vom proximalen Ende haben und die Umfangslage aus vorgegebenen Markierungen am proximalen Ende des Verriegelungsnagels, welche bewirken. daß das Zielgerät in vorgegebener Drehlage mit dem Nagel verbunden wird. Wie jedoch oben ausgeführt, ist eine genaue Lageidentifizierung der Querbohrungen nur auf mechanischem Wege mit den bekannten Mitteln nicht möglich.

Der Erfindung liegt die Aufgabe zugrunde, einen Verriegelungsnagel zu schaffen, mit dem möglich ist, auch ohne Durchleuchtungsmittel auf einfache Weise die Lage der distalen Querbohrungen zu bestimmen.

Diese Aufgabe wird durch die Merkmale des Patentanspruch 1 gelöst.

Bei der Erfindung ist eine achsparallele Nut in die Außenseite des Nagels eingeformt. Sie erstreckt sich zu beiden Seiten zumindest derjenigen Querbohrung, die dem distalen Ende des Nagels nächstgelegen ist. Die Längsachse der Nut schneidet die Achse der Bohrung annähernd senkrecht. Die Nut ist nach einer Ausgestaltung der Erfindung im Querschnitt vorzugsweise U-förmig, wobei sie vorzugsweise im Grund gerundet ist. Vorzugsweise erstreckt sich die Nut über beide Querbohrungen nach distal und proximal hinaus. Die maximale Breite der Nut kann kleiner als der Durchmesser der

Querbohrungen sein. Vorzugsweise ist das distale Ende des Verriegelungsnagels zylindrisch geformt. Das Ende kann wahlweise hohl oder massiv sein.

Herkömmliche Zielgeräte werden am proximalen Ende des Verriegelungsnagels axial und bezüglich ihrer relativen Drehlage festgelegt. Die Lage der äußersten distalen Querbohrung läßt sich daher auch ungefähr vorgeben. Dies kann bei dem erfindungsgemäßen Verriegelungsnagel in gleicher Weise erfolgen. Nachdem die ungefähre Lage gefunden worden ist, kann nun nach Stichinzision der Knochen kortikal aufgebohrt werden. Es wird jedoch nur die zugeordnete Knochenwand aufgebohrt. Anschließend wird ein im Durchmesser kleinerer Bohrer oder ein Drahtstift oder dergleichen durch die Knochenbohrung hindurchgeführt. Der Operateur kann nun ertasten, ob der Stift ohne weiteres weiter eingeführt werden kann, indem er durch die distale Querbohrung hindurchgeführt wird. Ist dies nicht der Fall, kann er feststellen, ob er mit dem inneren Ende des Stiftes in die Nut gelangt ist. Er kann dies insbesondere dadurch feststellen, daß er mit Hilfe des noch am Nagel angebrachten Zielgerätes den Verriegelungsnagel ein wenig verdreht. Dadurch kann er die Drehlage des Verriegelungsnagels im Hinblick auf die bereits vorgenommene Knochenbohrung justieren. Um nun auch in axialer Richtung eine Ausrichtung der Querbohrung zur Knochenbohrung zu erhalten, wird mit Hilfe des Zielgerätes der Nagel entweder ein wenig weiter eingeschlagen oder etwa herausgezogen, bis die richtige Lage der Querbohrung eingestellt ist.

Es ist sehr unwahrscheinlich, daß der Verriegelungsnagel in dem Bereich zwischen den beiden distalen Querbohrungen so verformt ist, daß die ursprüngliche Relativlage beider Querbohrungen verändert wird. Wenn daher die erste Querbohrung in ihrer Lage bestimmt worden ist, liegt damit auch die Lage der zweiten Bohrung fest. Mit dem erfindungsgemäßen Verriegelungsnagel läßt sich daher auf einfache Weise ohne Zuhilfenahme von Durchleuchtungsmitteln eine Verschraubung des distalen Endes des Verriegelungsnagels vornehmen.

Um jedoch auch die zweite Querbohrung im Verriegelungsnagel ohne weiteres aufzufinden, damit an der richtigen Stelle die zweite Knochenbohrung vorgenommen werden kann, sieht die Erfindung ein geeignetes Zielgerät vor. Es weist einen Griffabschnitt auf, der in herkömmlicher Weise mit dem proximalen Ende des Verriegelungsnagels verbindbar ist, um es an den Verriegelungsnagel in axialer und Drehrichtung festzulegen. Mit dem Griffabschnitt ist auch ein Zielstab verbindbar, der am Griffabschnitt lösbar anbringbar ist, jedoch im angebrachten Zustand axial und drehstabil festgelegt ist. Der Zielstab erstreckt sich parallel und im Abstand zum Verriegelungsnagel, wenn letzterer am Griffabschnitt angebracht ist. Der Zielstab erstreckt sich mithin extern parallel zu dem Gliedmaß, dessen Knochen versorgt werden soll. Distal weist der Zielstab zwei Querbohrungen auf, deren Abstand den Querbohrungen des Verriegelungsnagels entspricht. Sie sind jedoch im Durchmesser größer, wie an sich bekannt, um eine Bohr- oder Zielhülse aufnehmen zu können. Erfindungswesentlich ist jedoch, daß der distale Abschnitt des Zielstabs aus einem elastisch nachgebenden Material besteht, zum Beispiel aus einem entsprechenden Kunststoff, vorzugsweise PTFE.

Der distale Abschnitt ist vorzugsweise zylindrisch geformt. Der proximale Teil des Zielstabs besteht hingegen aus einem relativ starren Material, beispielsweise aus Metall, um eine drehstabile Aufnahme im Griffabschnitt zu ermöglichen.

Wird bei dem oben beschriebenen Verfahren eine Knochenschraube in die am weitesten distal liegende Querbohrung des Verriegelungsnagels eingeschraubt, kann anschließend der distale Stab auf den noch mit der Knochenschraube verbundenen Schaft des Schraubers aufgesteckt werden. Das proximale Ende des Zielstabs wird jedoch in vorgegebener Drehlage mit dem Griffabschnitt des Zielgerätes verbunden. Hat beim Verriegelungsnagel beim Eintreiben eine Torsion stattgefunden, ergibt sich automatisch auch eine Torsion des Zielstabes. Diese Torsion wird sich vor allen Dingen im elastisch nachgebenden distalen Abschnitt abspielen, was zur Folge hat, daß damit auch die zweite Querbohrung des Zielstabs nunmehr präziser zur zweiten Querbohrung des Verriegelungsnagels ausgerichtet ist. Ähnliches ist der Fall, wenn der Nagel im Knochen gebogen wurde. Der distale Stababschnitt erleidet dann ebenfalls eine Verbiegung. Es ist daher möglich, mit Hilfe der zweiten Querbohrung des distalen Abschnitts nunmehr auch die zweite Knochenbohrung vorzunehmen und anschließend die zweite Knochenschraube einzusetzen.

Die Erfindung wird nachfolgend anhand von Zeichnungen näher erläutert.
- Fig.: 1 zeigt äußerst schematisch perspektivisch ein Zielgerät nach der Erfindung und einen Knochennagel in einem schematisch angedeuteten Röhrenknochen.
- Fig. 2: zeigt äußerst schematisch perspektivisch das Zielgerät nach Fig. 1 in vollständiger Gestalt.
- Fig. 3: zeigt eine Seitenansicht des Zielstabs des Zielgeräts nach den Fig. 1 und 2.
- Fig. 4: zeigt eine Seitenansicht des gegenüber den Fig. 1 und 2 abgewandelten Griffabschnitts des Zielgeräts.
- Fig. 5: zeigt einen Teil des Griffabschnitts nach Fig. 4 in der Seitenansicht gemäß Pfeil 5.
- Fig. 6: zeigt einen Schnitt durch die Darstellung nach Fig. 5 entlang der Linie 6-6.
- Fig. 7: zeigt einen Schnitt durch den Zielstab nach Fig. 3 entlang der Linie 7-7.
- Fig. 8: zeigt das distale Ende des Verriegelungsnagels nach der Erfindung.
- Fig. 9: zeigt einen Schnitt durch den Nagelabschnitt nach Fig. 8 entlang der Linie 9-9.
- Fig. 10: zeigt einen Schnitt durch den Nagelabschnitt nach Fig. 8 entlang der Linie 10-10.

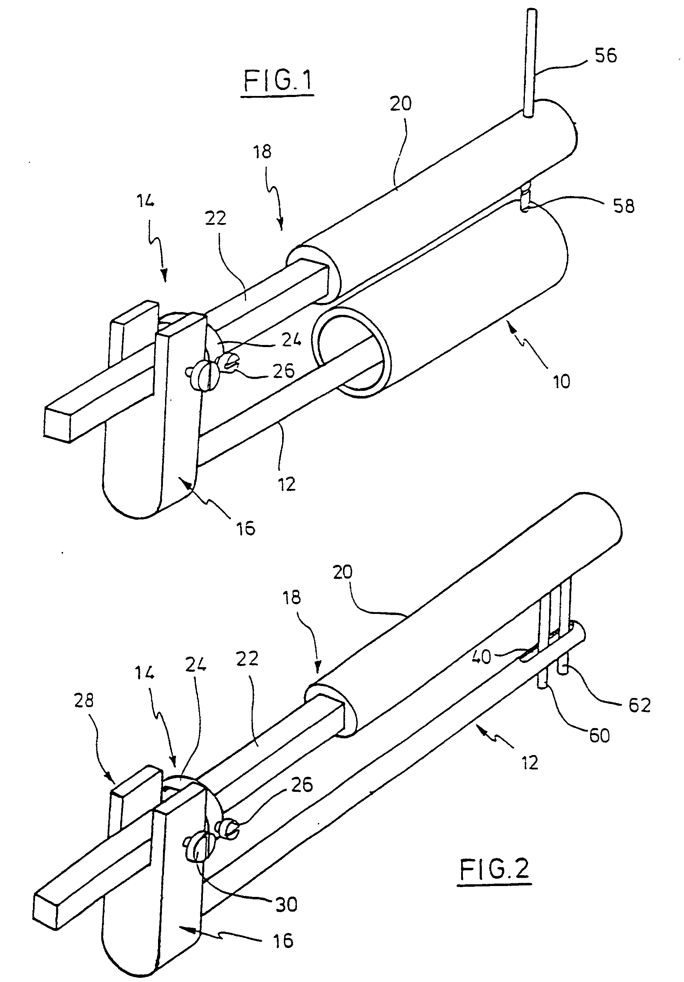

In Fig. 1 ist ein Röhrenknochen 10 angedeutet, der mit Hilfe eines auch nur angedeutet gezeichneten Verriegelungsnagels 12 versorgt werden soll. Ein Zielgerät 14, das in Fig. 1 angedeutet ist, weist einen Griffabschnitt 16 auf, mit dem das proximale Ende des Nagels 12 axial und drehfest fest verbunden ist. Die Art der Verbindung ist nicht dargestellt. Sie ist an sich bekannt. Parallel zum Nagel 12 erstreckt sich ein Zielstab 18, der aus einem distalen Abschnitt 20 und einem proximalen Abschnitt 22 besteht. Der proximale Abschnitt 22 ist ein Vierkant aus Metall. Der distale Abschnitt 20 ist zylindrisch und aus einem elastischen Kunststoffmaterial, zum Beispiel PTFE, geformt. Die Abschnitte 20, 22 sind in geeigneter, jedoch nicht dargestellter Weise miteinander verbunden. Auf dem Abschnitt 22 des Zielstabs 18 sitzt drehfest ein Feststellring 24, der jedoch axial verschiebbar ist, wobei die axiale Lage mit Hilfe einer Feststellschraube 26 festgestellt werden kann. Der Griffabschnitt 16 weist einen gegabelten Abschnitt 28 auf, wobei der Spalt zwischen den Schenkeln so bemessen ist, daß der Vierkantabschnitt 22 passend aufgenommen ist. Eine weitere Feststellschraube 30 dient dazu, den Vierkantabschnitt 22 im Gabelabschnitt 28 festzulegen.

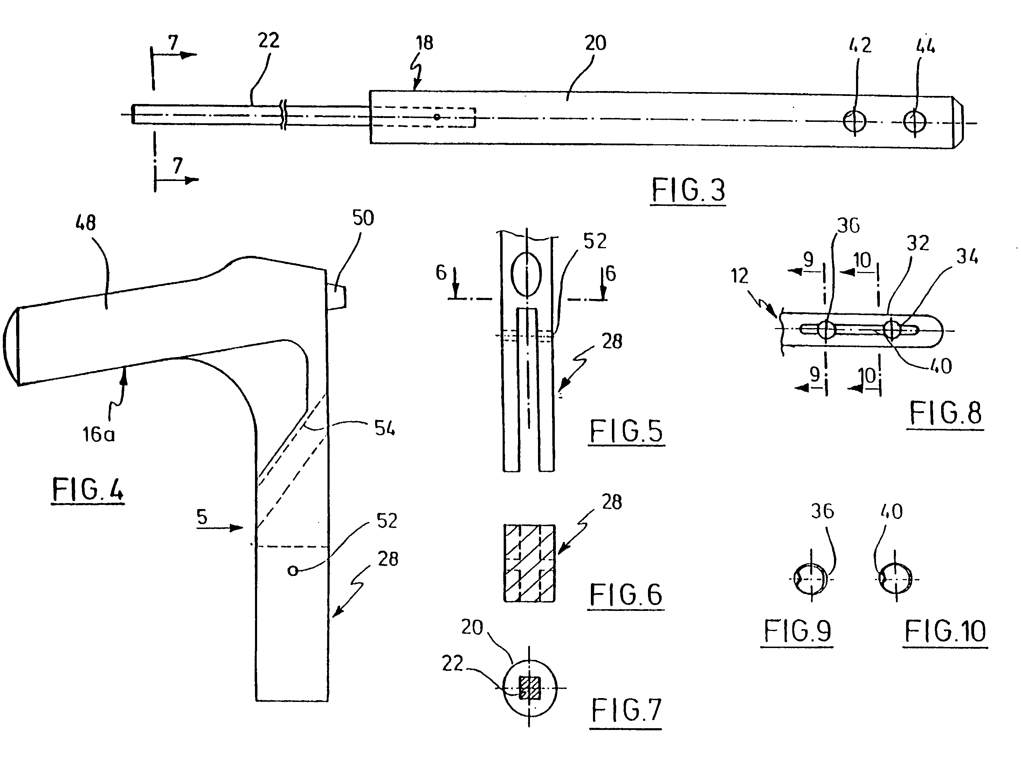

Wie aus Fig. 2 hervorgeht, ist der Verriegelungsnagel 12 mit zwei distalen Querbohrungen versehen, wie an sich bekannt. Dies geht deutlicher aus der Darstellung nach Fig. 8 hervor. Der distale Abschnitt des Verriegelungsnagels ist mit 32 bezeichnet und die Querbohrungen haben die Bezugszeichen 34 und 36. Aus der Betrachtung der Fign. 2 und 8 bis 10 geht hervor, daß außen im distalen Abschnitt 32 eine Nut 40 geformt ist. die achsparallel verläuft und sich über die Bohrungen 34, 36 hinaus jeweils nach distal bzw. proximal erstreckt. Die Erstreckung beidseits der Bohrungen 34, 36 beträgt z.B. 5 mm. Die Nut 40 erstreckt sich außerdem bis in die Bohrungen 34, 36 hinein. Aus den Fig. 9 und 10 geht hervor, daß die Nut annähernd U-förmigen Querschnitt aufweist und im Grund gerundet ist. Sie hat eine etwas geringere Breite als der Durchmesser der Querbohrungen 34, 36.

In den Fign. 3 und 7 ist etwas deutlicher der Zielstab 18 dargestellt. Man erkennt zum Beispiel, daß der Vierkantabschnitt 22 zum Teil in das Material des distalen Abschnitts 20 eingebettet ist. Distal weist der distale Abschnitt 20 zwei Querbohrungen 42, 44 auf. Der Abstand ihrer Achsen entspricht dem Abstand der Achsen der Querbohrungen 36, 34 (die Zeichnungen sind nicht maßstabgerecht). Der Durchmesser der Querbohrungen 42, 44 ist größer als der Durchmesser der Querbohrungen 34, 36, da sie auch eine Hülse aufnehmen müssen, über welche ein Bohrer eingeführt werden kann, worauf weiter unten noch näher eingegangen wird.

Der Griffabschnitt 16a nach Fig. 4 unterscheidet sich von dem nach Fig. 1 und 2 darin, daß er winkelförmig ist. Er weist am oberen rechten Ende des Griffes 48 einen Konus 50 auf, auf den der nicht gezeigte Verriegelungsnagel aufgeschoben wird. Die übrige Befestigung des Verriegelungsnagels wird, wie schon erwähnt, nicht beschrieben.

Der Griffabschnitt 16a weist ebenso wie der Griffabschnitt 16 einen Gabelabschnitt 28 auf zur passenden Aufnahme des Vierkantabschnitts 22. In Fig. 5 ist eine Gewindequerdurchbohrung 52 zu erkennen zur Aufnahme der Feststellschraube 30 nach Fig. 2.

Oberhalb des Gabelabschnitts 28 weist der Griffabschnitt 16a eine schräge Durchbohrung 54 auf, auf deren Funktion nicht eingegangen wird.

Beim Implantieren und anschließenden Auffinden der Querdurchbohrungen 34, 36 des Verriegelungsnagels 12 ist der Abstand dieser Querdurchbohrungen vom proximalen Ende für den noch nicht eingetriebenen Nagel bekannt. Daher kann der Zielstab 18 in seiner axialen Lage so befestigt werden, daß seine Querdurchbohrungen 42, 44 zur vermuteten Lage des implantierten Verriegelungsnagels bzw. seiner Querbohrungen 34, 36 ausgerichtet sind. In Wirklichkeit kann jedoch durch ein Biegen des Verriegelungsnagels 12 oder durch eine Torsion die tatsächliche Lage der Querbohrungen 34, 36 eine abweichende sein. In jedem Fall wird bei dem Zielvorgang zunächst von der vermuteten Lage ausgegangen. In dieser wird dann mit Hilfe eines Bohrers 56 gemäß Fig. 1 in den Knochen 10 eine Bohrung 58 vorgenommen, und zwar lediglich in der zugeordneten Knochenwand. Anschließend wird der Zielstab 18 entfernt. Danach führt der Operateur einen Bohrer von kleinerem Durchmesser (vorher hat er vielleicht einen 5,5 mm Bohrer verwendet und der nachfolgende Bohrer und der Stift haben einen Durchmesser von 3,5 mm) von Hand durch die Knochenbohrung ein, bis er den Nagel erfühlt. Falls der Operateur die Querbohrung 34 nicht erfühlt, wird der Nagel 12 leicht gedreht, bis der Operateur die Nut 40 ertastet. Danach wird der Nagel 12 vor und zurück bewegt, bis sich der Bohrerschaft bzw. der Drahtstift leicht weiter einführen läßt. Danach kann auch die gegenüberliegende Kortikalis gebohrt und anschließend die Knochenschraube eingeschraubt werden. Der Schaft des Schraubendrehers verbleibt an der Schraube, und der Zielstab 18 wird danach in den Griffabschnitt 16 bzw. 16a in reproduzierbarer Lage festgelegt. Dabei wird der distale Abschnitt 20 gegebenenfalls tordiert bzw. verformt, wenn auch der Nagel beim Eintreiben eine Verformung bzw. Torsion erlitten hat. Die zweite Querbohrung 42 des distalen Abschnitts 20 ist dann exakt zur zweiten Querbohrung 36 des Nagels 12 ausgerichtet, so daß nunmehr auch die Kortikalis für die zweite Querbohrung 36 gebohrt werden kann zum anschließenden Verschrauben mit Hilfe der nicht gezeigten zweiten Knochenschraube.

In Fig. 2 soll mit Hilfe der dargestellten Stifte 60, 62 lediglich das Zusammenwirken von Zielstab 18 und Querbohrungen 34, 36 des Nagels 12 angedeutet werden.

Anschließend kann auch proximal der Verriegelungsnagel 12 auf an sich bekannte Weise verriegelt werden. Es ist unwahrscheinlich, daß in diesem Bereich eine Verformung stattgefunden hat. Daher stimmt eine am Zielstab 18 vorgesehene Bohrung (nicht gezeigt) in jedem Fall mit der proximalen Querbohrung des Nagels 12 überein. Hier ergeben sich mithin für den Operateur keine Schwierigkeiten.

## Patentansprüche

1. Implantationssystem mit
- einem Verriegelungsnagel (12) zur Versorgung von Frakturen von Röhrenknochen, der am distalen Ende distal beabstandete, parallele Querbohrungen (34, 36) und am proximalen Ende mindestens eine weitere Querbohrung aufweist zur Aufnahme von Knochenschrauben, wobei im distalen Abschnitt (32) des Verriegelungsnagels (12) eine achsparallele Nut (40) in die Außenseite des Nagels (12) eingeformt ist, die sich zu beiden Seiten der Querbohrung (34), in diese übergehend, erstreckt, jedoch nur ein wenig distal über die dem distalen Ende nächstgelegene Querbohrung (34) und nur ein wenig proximal über die dem proximalen Ende des Nagels nächstgelegene distale Querbohrung, wobei die Längsachse der Nut (40) die Achse der Querbohrungen (34, 36) annähernd schneidet, die Nut im Querschnitt U-förmig ist und ihre maximale Breite kleiner ist als der Durchmesser der Querbohrungen (34, 36),
- einem Zielgerät (14) für den Verriegelungsnagel (12), der einen Griffabschnitt (16, 16a) aufweist mit Mitteln zur Festlegung des proximalen Endes des Verriegelungsnagels (12) in axialer und Drehrichtung sowie einen Zielabschnitt (18), der an einem Ende axial und drehfest am Griffabschnitt (16, 16a) befestigt ist, der sich annähernd parallel zum und im Abstand vom eingespannten Verriegelungsnagel (12) erstreckt und am distalen Ende Querbohrungen aufweist, die zu den Querbohrungen (34, 36) im Verriegelungsnagel (12) ausrichtbar sind,
- einem durch eine Querbohrung des Zielabschnitts (18) hindurchführbaren Bohrer (56) zum Aufbohren der nächstgelegenen Wand des Knochens und
- einem Stift oder einem Bohrer mit kleinerem Durchmesser als der erste Bohrer zum Ertasten der Nut (40) und einer der Querbohrungen (34, 36) des Verriegelungsnagels (12).

2. Implantationssystem nach Anspruch 1, **dadurch gekennzeichnet, daß** der distale Abschnitt (32) des Nagels (12) zylindrisch geformt ist.

3. Implantationssystem nach Anspruch 1 oder 2, **dadurch gekennzeichnet, daß** die Nut (40) sich etwa 5 mm über die Querbohrungen (34, 36) hinaus nach distal und proximal erstreckt.

## Claims

1. An implantation system comprising:
- A locking nail (12) to care for fractures in cylindrical hollow bones wherein two axially spaced, parallel cross-bores (34, 36) are provided at the distal end and at least another cross-bore is provided at the proximal end for the reception of bone screws, wherein in the distal portion (32) of the locking nail (12) an axially parallel groove (40) is formed in the outside of the nail (12) and extends at either side of the cross-bores (34) and opens into it, but only in a slightly distal manner across the cross-bore (34) positioned nearest to the distal end and only in a slightly proximal manner across the distal cross-bore (36) positioned nearest to the proximal end of the nail, wherein the longitudinal axis of the groove (40) approximately intersects the axis of the cross-bore (34, 36), the groove being U-shaped in cross-section, and its maximum width is smaller than the diameter of the cross-bores (34, 36),
- a targeting apparatus (14) for the locking nail (12), comprising:a handle portion (16, 16a) which has means for locating the proximal end of the locking nail (12) in the axial and rotational directions and an aim-taking portion (18) which is mounted on the handle portion (16, 16a) at one end in an axially and rotationally stable way, which extends in parallel with and at a distance from the clamped locking nail (12), and has cross-bores (36) at the distal end, which can be aligned with the cross-bores (34, 36) in the locking nail (12),
- a drill (56) adapted to be extended through one cross-bore of portion (18) for drilling of the adjacent wall of the bore and
- a pin or drill having a smaller diameter than that of the first drill for touch-feeling the grove (40) and one of the cross-bores (34, 36) of the locking nail.

2. The implantation system according to claim 1, **characterized in that** the distal portion (32) of the nail (12) is of a cylindrical shape.

3. The implantation system according to claim 1 or 2, **characterized in that** the groove (40) extends by about 5 mm beyond the cross-bores (34, 36) in the distal and proximal direction.

## Revendications

1. Système d'implantation comportant :
- une broche de verrouillage (12) pour le traitement de fractures d'os tubulaires, qui comporte à l'extrémité distale des alésages transversaux (34, 36) parallèles espacés en direction distale et, à l'extrémité proximale, au moins un autre alésage transversal pour la réception de vis pour os, une rainure (40) parallèle à l'axe étant formée dans la partie distale (32) de la broche de verrouillage (12) dans le côté extérieur de la broche (12) qui s'étend des deux côtés de l'alésage transversal (34) en le traversant, mais seulement un peu en direction distale par rapport à l'alésage transversal (34) le plus proche de l'extrémité distale et seulement un peu en direction proximale par rapport à l'alésage transversal distal le plus proche de l'extrémité proximale de la broche, l'axe longitudinal de la rainure (40) coupant approximativement l'axe des alésages transversaux (34, 36), la rainure ayant une section transversale en forme de U et sa largeur maximale étant plus petite que le diamètre des alésages transversaux (34, 36),
- un appareil de visée (14) pour la broche de verrouillage (12), qui comporte une partie de préhension (16, 16a) avec des moyens pour la fixation de l'extrémité proximale de la broche de verrouillage (12) dans la direction axiale et de rotation, ainsi qu'une partie de visée (18), qui est fixée de manière fixe axialement et en rotation par une extrémité à la partie de préhension (16, 16a), qui s'étend à peu près parallèlement et à distance de la broche de verrouillage (12) serrée et comporte à l'extrémité distale des alésages transversaux qui peuvent être alignés avec les alésages transversaux (34, 36) dans la broche de verrouillage (12),
- un foret (56) qui peut être guidé à travers un alésage transversal de la partie de visée (18) pour aléser la paroi la plus proche de l'os et
- une pointe ou un foret de plus petit diamètre que le premier foret pour palper la rainure (40) et l'un des alésages transversaux (34, 36) de la broche de verrouillage (12).

2. Système d'implantation selon la revendication 1, **caractérisée en ce que** la partie distale (32) de la broche (12) a une forme cylindrique.

3. Système d'implantation selon la revendication 1 ou 2, **caractérisée en ce que** la rainure (40) dépasse d'environ 5 mm des alésages transversaux (34, 36) en direction distale et en direction proximale.
